(19)

Europäisches
Patentamt

European
Patent Office

Office européen
des brevets

(11) **EP 4 520 840 A1**

(12) **EUROPEAN PATENT APPLICATION**

(43) Date of publication:
**12.03.2025 Bulletin 2025/11**

(21) Application number: **23195471.0**

(22) Date of filing: **05.09.2023**

(51) International Patent Classification (IPC):
*C12Q 1/6806* (2018.01)    *C12Q 1/6855* (2018.01)

(52) Cooperative Patent Classification (CPC):
(C-Sets available)
**C12Q 1/6855; C12Q 1/6806**    (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Fraunhofer-Gesellschaft zur Förderung der angewandten Forschung e.V.
80686 München (DE)**

(72) Inventors:
• **ELGETI, Vanessa Kimerly
70569 Stuttgart (DE)**

• **SONNTAG, Mirko
70569 Stuttgart (DE)**
• **SOHN, Kai
70569 Stuttgart (DE)**
• **JENNER, Lucca
70569 Stuttgart (DE)**

(74) Representative: **Altmann Stößel Dick
Patentanwälte PartG mbB
Theodor-Heuss-Anlage 2
68165 Mannheim (DE)**

Remarks:
The complete document including Reference Table(s) and the Sequence Listing(s) can be downloaded from the EPO website

(54) **SUPPRESSION PCR-BASED SELECTIVE ENRICHMENT SEQUENCING**

(57)    The present invention relates to a method for providing at least one detection template for detecting a marker sequence in a sample comprising template DNA, said method comprising (a) providing a plurality of elongation templates having a stem-loop structure from said template DNA; (b) providing at least one oligonucleotide (fusion primer) comprising a 5' universal sequence and a 3' targeting sequence; (c) incubating said plurality of elongation templates of step (a) with the least one fusion primer of step (b) under conditions (i) allowing hybridization of said at least one fusion primer to at least one target nucleic acid sequence in an elongation template, if present, and (ii) causing elongation along the elongation template of any fusion primer hybridized to an elongation template; (d) removing the fusion primer from the reaction products of step (c); and (e) thereby providing a detection template for detecting a marker sequence in a sample. The present invention also relates to a method for determining at least one marker sequence in a sample and to a method for diagnosing disease in a sample of a subject, as well as compositions of matter, devices, and kits related thereto.

EP 4 520 840 A1

(52) Cooperative Patent Classification (CPC): (Cont.)

C-Sets
**C12Q 1/6806, C12Q 2525/155, C12Q 2525/161,
C12Q 2525/301, C12Q 2531/101, C12Q 2549/119;
C12Q 1/6855, C12Q 2525/155, C12Q 2525/161,
C12Q 2525/301, C12Q 2531/101, C12Q 2549/119**

**Description**

The present invention relates to a method for providing at least one detection template for detecting a marker sequence in a sample comprising template DNA, said method comprising (a) providing a plurality of elongation templates having a stem-loop structure from said template DNA; (b) providing at least one oligonucleotide (fusion primer) comprising a 5' universal sequence and a 3' targeting sequence; (c) incubating said plurality of elongation templates of step (a) with the least one fusion primer of step (b) under conditions (i) allowing hybridization of said at least one fusion primer to at least one target nucleic acid sequence in an elongation template, if present, and (ii) causing elongation along the elongation template of any fusion primer hybridized to an elongation template; (d) removing the fusion primer from the reaction products of step (c); and (e) thereby providing a detection template for detecting a marker sequence in a sample. The present invention also relates to a method for determining at least one marker sequence in a sample and to a method for diagnosing disease in a sample of a subject, as well as compositions of matter, devices, and kits related thereto.

Sepsis is a life-threatening illness that can occur when the whole body reacts to an infection. Despite intensive research, sepsis remains the third leading cause of mortality in intensive care units. Pathophysiologically, during sepsis progression there is an initial hyper-inflammatory phase which provokes the onset of a hypo-inflammatory stage, partly occurring in parallel. Frequently, disease severity is already far advanced when sepsis is diagnosed in the patient and liver damage, a relatively late event during sepsis progression, has already occurred. During sepsis, organ failure, often followed by a multi-organ-dysfunction syndrome (MODS), frequently results in the patient's death.

Causative for sepsis are infections with microorganisms, mostly bacteria but also viral, fungal and parasite-derived sepsis are described in literature (Eykyn et al. (1990) J. Antimicrob. Chemother 25, 41-58; Dolin al. (2019) Microbiol. Insights 12, 117863611882508). The most commonly found bacteria in septic patients are *Staphylococcus aureus, Streptococcus pneumoniae, Enterococcus spp., Escherichia coli, Klebsiella spp. or Pseudomonas aeruginosa.*

Besides bacteria, also fungi are commonly isolated in septic patients, especially *Candida spp.* like *Candida albicans* or in some cases also *Aspergillus spp.* Infection sites for causative agents are typically the respiratory tract, the urinary tract and the abdomen. Also, wounds or device-related entry sites are described for sepsis-causing pathogens (Mayr et al. (2010) JAMA 303, 2495-2503).

To quantify and qualify the patient's outcome and sepsis severity, clinical prognostic parameters are used to define the health state of septic patients. Commonly applied scoring systems are the SOFA (Sequential (Sepsis-related) Organ Failure Assessment), LODS (Logistic Organ Dysfunction Score), SIRS (Systemic Inflammatory Response Syndrome) or NEWS (National Early Warning System). In addition to patient evaluation via scoring systems, the identification of causative pathogens is crucial. Specific and adequate treatment at an early time point increases the survival chances of a septic patient (Seymour et al. (2017) N. Engl. J. Med 376, 2235-2244). Currently, blood culture analysis is the standard method in clinics to diagnose causative pathogens (Patel, M. (2016) J. Acad. Clin. Microbiol. 18, 74). Blood samples are cultivated on different cultivation media for several days until a positive identification is obtained. This method, however, has limitations and drawbacks, e.g. blood culture is rather time-consuming with testing times up to five days. Furthermore, only a low specificity and sensitivity is obtained because of several factors: not all infective pathogens can be detected and the procedure is prone to contaminations, which can lead to false-positive signals (Murray & Masur (2012) Crit. Care Med. 40,3277-3282). Also, antibiotic treatment can negatively affect the detection of pathogens. In addition, not all pathogens can be detected by this method, especially viruses or parasites, and even for many bacteria, blood culture is not a suitable diagnostic method. This suggests that blood culture is of limited value for fast, sensitive and specific detection of causative agents of sepsis.

First discovered in 1948 by Mandel & Métais ((1948) Comptes Rendus des Seances la Soc. Biol. ses Fil. 142), cell-free DNA (cfDNA) has stepped into the scientific spotlight in recent years for the analysis and molecular profiling in diagnosis, prognosis and therapy (reviewed e.g. in Aucamp et al. (2018) Biol. Rev. 93, 1649-1683). All nucleated cells in the body release DNA - endogenous cells as well as microorganisms, malignant cells or damaged tissue. Several aspects like apoptosis, necrosis, inflammation, exercise and even aging are stimuli for DNA release. This means that cfDNA release is not only a feature of cellular breakdown mechanisms like apoptosis, autophagy or necrosis. In addition, living cells actively release DNA for various reasons.

Polymerase chain reaction (PCR) has long been used for amplification of DNA, e.g. in diagnostic settings. Although a powerful tool, PCR in diagnostics frequently is limited by a low ratio of target DNA to total DNA in samples, thus requiring measures to limit amplification of undesired DNA (unspecific amplification). One of such measures is referred to as "suppression PCR" in the literature (Broude et al., (1999) Genet. Anal. 15, 51-63; Broude et al. (2001) Proc. Natl. Acad. Sci. U. S. A. 98, 206-211; US 2004/0126760 A1). By ligating adapters with high melting temperatures to DNA templates, the DNA templates can be caused to form stem-loop structures, which prevent amplification of templates with adapter primers only. However, suppression PCR uses an adapter-specific first primer and a target specific second primer, which may cause unwanted bias for some target sequences in multiplex settings.

In accordance, the present invention relates to a method for providing at least one detection template for detecting a marker sequence in a sample comprising template DNA, said method comprising

(a) providing a plurality of elongation templates having a stem-loop structure from said template DNA;

(b) providing at least one oligonucleotide (fusion primer) comprising a 5' universal sequence and a 3' targeting sequence;

(c) incubating said plurality of elongation templates of step (a) with the least one fusion primer of step (b) under conditions (i) allowing hybridization of said at least one fusion primer to at least one target nucleic acid sequence in an elongation template, if present, and (ii) causing elongation along the elongation template of any fusion primer hybridized to an elongation template;

(d) removing the fusion primer from the reaction products of step (c); and

(e) thereby providing a detection template for detecting a marker sequence in a sample.

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. identifying "a cell" relates to identifying at least one cell, preferably to identifying a plurality of cells. The term "plurality" is used herein in its common meaning to relate to a quantity of more than one, i.e. at least two. Thus, a plurality may preferably be a number of two or more, three or more, five or more, ten or more, 25 or more, 50 or more 100 or more, 1000 or more.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are in vitro methods. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned herein.

As used herein, if not otherwise indicated, the term "about" relates to the indicated value with the commonly accepted technical precision in the relevant field, preferably relates to the indicated value $\pm$ 20%, more preferably $\pm$ 10%, most preferably $\pm$ 5%. Further, the term "essentially" indicates that deviations having influence on the indicated result or use are absent, i.e. potential deviations do not cause the indicated result to deviate by more than $\pm$ 20%, more preferably $\pm$ 10%, most preferably $\pm$ 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, buffer, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

The term "polynucleotide", as used herein, refers to a linear or circular nucleic acid molecule. The polynucleotide of the present invention shall be provided, preferably, either as an isolated polynucleotide (i.e. isolated from its natural context) or in genetically modified form, preferably comprising at least one heterologous sequence. The term encompasses single- as well as, partially or completely, double-stranded polynucleotides. Preferably, the polynucleotide is a DNA polynucleotide, which may also be referred to as "DNA". Moreover, comprised are also chemically modified polynucleotides including naturally occurring modified polynucleotides such as glycosylated or methylated polynucleotides or artificially modified derivatives such as biotinylated polynucleotides, locked nucleic acids, peptide nucleic acids, and the like. In view of the description herein, template DNA, elongation templates, detection templates, fusion primers, adapter molecules, universal sequence primers, and adapter primers preferably are polynucleotides.

Unless specifically indicated otherwise, reference to specific polynucleotides herein preferably includes polynucleotide variants. The term "polynucleotide variant", as used herein, relates to a variant of a polynucleotide referred to herein comprising a nucleic acid sequence characterized in that the sequence can be derived from the aforementioned specific nucleic acid sequence by at least one nucleotide substitution, addition and/or deletion, wherein the polynucleotide variant shall have the function and/or activity as specified for the specific polynucleotide. Thus, a variant of a marker sequence may e.g. be a sequence of an ortholog, a paralog, or another homolog of the specific polynucleotide. Also preferably, said polynucleotide variant is or is derived from a non-naturally occurring allele of the specific polynucleotide. Polynucleotide variants also encompass polynucleotides comprising a nucleic acid sequence which is capable of hybridizing to the aforementioned specific polynucleotides, preferably, under stringent hybridization conditions. These stringent conditions are known to the skilled worker and can be found e.g. in Current Protocols in Molecular Biology, John Wiley & Sons, N. Y. (1989), 6.3.1-6.3.6. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. As referred to herein, "hybridizing specifically" preferably means hybridizing under stringent conditions, more preferably means hybridizing to the target sequence compared to a non-target sequence by a factor at least 2fold, preferably at least 5fold, more preferably at least 10fold, even more preferably at least 100fold. Thus, a primer or probe specifically hybridizing to a target sequence provides for a specific amplification and/or a specific signal. Further polynucleotide variants include polynucleotides comprising nucleic acid sequences which are at least 70%, preferably at least 80%, more preferably at least 90%, even more preferably at least 95%, still more preferably at least 98%, most preferably at least 99%, identical to the specifically indicated nucleic acid sequences. The percent identity values are, preferably, calculated over the entire nucleic acid sequence region, preferably as specified herein elsewhere. The polynucleotides of the present invention either consist, essentially consist of, or comprise the aforementioned nucleic acid sequences. Thus, they may contain further nucleic acid sequences as well.

The term "fragment" of a biological macromolecule, preferably of a polynucleotide, is used herein in a wide sense relating to any sub-part, preferably subdomain, of the respective biological macromolecule comprising the indicated sequence, structure and/or function. Thus, the term includes sub-parts generated by actual fragmentation of a biological macromolecule, but also sub-parts derived from the respective biological macromolecule in an abstract manner, e.g. in *silico.* In accordance, the term "subsequence" of a biological sequence, preferably of a nucleic acid sequence or of an amino acid sequence, relates to any sub-part of the respective biological sequence comprising the indicated sequence and preferably having the indicated function, if indicated.

Unless specifically indicated otherwise herein, the compounds specified, in particular the polynucleotides, may be comprised in larger structures, e.g. may be covalently or non-covalently linked to further sequences and/or auxiliary groups, such as detection agents, e.g. a dye, chemical groups enabling binding to a solid surface, and the like. In particular, polynucleotides, e.g. DNA, as specified may be comprised in fusion polynucleotides comprising further polynucleotides, which may serve e.g. as a tag for purification and/or detection, as a linker, or to extend the in vivo half-life of a compound. The term "tag" refers to a pre-determined nucleic acid sequence which may be added to or introduced into the polynucleotide, e.g. functioning as a barcode.

The term "polypeptide", as used herein, refers to a molecule consisting of several, typically at least 20 amino acids that are covalently linked to each other by peptide bonds. Molecules consisting of less than 20 amino acids covalently linked by peptide bonds are usually considered to be "peptides". Preferably, the polypeptide comprises of from 50 to 1000, more preferably of from 75 to 1000, still more preferably of from 100 to 500, most preferably of from 110 to 400 amino acids. Preferably, the polypeptide is comprised in a fusion polypeptide and/or a polypeptide complex.

The degree of identity (e.g. expressed as "%identity") between two biological sequences, preferably DNA, RNA, or amino acid sequences, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polynucleotide or polypeptide, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (e.g. BLAST, GAP, BESTFIT, PASTA, or TFASTA), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. More preferably, the Basic Local Alignment Search Tool (BLAST) implementation is used with default parameter values for alignment. In the context of biological sequences referred to herein, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "sample", as used herein, refers to any composition of matter known or suspected to comprise a template DNA, preferably known or suspected to comprise a marker sequence to be detected. The sample may be of any state of matter. Thus, preferably, the sample is a liquid, semisolid, solid, or gaseous sample. Preferably, the sample is a liquid or semisolid sample, preferably an aqueous solution, which may optionally comprise other solvents, including organic solvents. The sample may also be a solid sample, e.g. comprising biological material. The sample may also be gaseous, e.g. an aerosol comprising liquid droplets or solid particles. Thus, the sample may also be a mixture of at least two materials, e.g. a solution, an emulsion, a foam, a sol, a foam, or a mixture thereof. The sample may be of any origin, e.g., it may be a sample of a naturally occurring composition of matter, such as a biological sample, or an artificial sample. A naturally occurring composition may in particular be a sample present in and/or withdrawn from the environment, i.e. an environmental sample. An artificial sample preferably includes any sample generated by human activity, such as a technical sample, e.g. an educt, an intermediate product, or a product of a technical process, a laboratory sample, and the like. It is to be understood that the sample may be further processed in order to carry out a method of the present invention. In particular, the sample may be processed to release template DNA, e.g. from cells or other material comprising DNA; also, template DNA may be enriched, e.g. by precipitation and/or by binding to a solid surface binding anionic compounds, e.g. magnetic beads binding DNA. Also preferably, cells or other particulate matter may be removed by centrifugation and/or filtration, and the like. Also, a subfraction of DNA in a sample may be isolated as template DNA, e.g. by size fractionation. Preferably, the aforesaid enrichment and/or subfractionation is non-sequence specific. As referred to herein, all types of pretreated samples are included in the term sample.

Preferably, the sample is a biological sample, the term "biological sample", as referred to herein, relating to any sample comprising biological material, preferably suspected or known to comprise template DNA; thus, the sample preferably is a sample obtained from a subject, is a food sample, an environmental sample, or the like. Preferably, the sample is a sample of a body fluid, a sample of separated cells, a sample from a tissue, including cancer tissue, or an organ, or a sample of wash/rinse fluid obtained from an outer or inner body surface of a subject. Preferably, the sample is a body fluid like blood, plasma, serum, urine, saliva, cerebrospinal fluid, lacrimal fluid, sputum, amniotic fluid, or pus. Also preferably, the sample is a tissue, preferably a sample from a site of infection or in fluid connection with a site of infection, or a cancer sample. Samples can be obtained by well-known techniques which include, preferably, scrapes, swabs or biopsies. Such samples can be obtained by use of brushes, (cotton) swabs, spatulas, rinse/wash devices, punch biopsy devices, puncture devices for cavities, such as needles or lances, or by other surgical instrumentation. Separated cells and/or cell-free liquids may be obtained from cell culture supernatants, body fluids, or the tissues or organs by separating techniques such as filtration, centrifugation, or cell sorting. In case of diagnosing disease, such as infection, the sample may in particular be a blood or blood-derived sample as specified herein below, a urine, cerebrospinal fluid, amniotic fluid, or a saliva sample. The biological sample may, however, also be a sample not directly taken from the body of a subject; as indicated herein above, a biological sample may be any sample comprising biological material. Thus, an environmental sample will typically be a biological sample. Also, samples comprising a mixture of biological material derived from a multitude of biological organisms, in particular wastewater, may be used as a sample.

The term "marker sequence", as used herein, relates to a nucleic acid sequence making possible identification of a biological organism, its properties, or a group thereof. Thus, the marker sequence may be unique to a biological organism. The marker sequence may, however, be typical for a group of organisms, e.g. a sequence comprised in a gene shared by a genus or a family of biological organisms. The marker sequence may also make identification of one or more properties of a biological organism possible; e.g. the marker sequence may be comprised in a resistance gene or a family of resistance genes such a beta-lactamases. Also, the marker sequence may allow identification of one or more of physiological properties of an organism, such as anaerobic growth, presence of pathogenicity factors, and the like. According to the method described herein, a multitude of marker sequences may be detected, preferably in the same detection reaction; thus, e.g. marker sequences indicative of family, genus, and/or species of a pathogenic microorganism, possible pathogenicity factors and antibiotic resistances, and/or metabolic properties of pathogenic microorganisms may be detected in parallel, preferably using the same elongation and/or amplification reactions and optionally the same detection reaction. Preferred marker sequences are described herein in the Examples.

The term "target sequence" is, in principle, understood by the skilled person. Preferably, the target sequence is a subsequence of a marker sequence which shall be detected (targeted). Depending on the length of the marker sequence, the target sequence may, however, also be the marker sequence. Preferably, the target sequence is selected to have a length suitable for hybridization with a primer oligonucleotide, in particular with a fusion primer, under the conditions applied in step (c). The skilled person can derive an appropriate target sequence from a marker sequence using tools commonly known in the art, e.g. melting point prediction tools and/or primer selection tools generally available. Preferably, the target sequence has a length of from 14 to 30 nucleotides, preferably of from 15 to 25 nucleotides, more preferably of from 16 to 24 nucleotides. The target sequence may, in principle, be any sequence comprised in a marker sequence; in case of marker sequences expected to be present at low numbers in a sample, the target sequence may be selected to have an increased probability of being present; e.g. in case cfDNA is used as a template DNA, the target sequence may be selected to be protected from degradation, e.g. by binding to a polypeptide such as a histone, a transcription factor, or the like. In view of the above, it is understood by the skilled person that the target sequence may be selected to be generic for a group of marker sequences, e.g. a group of resistance genes, a group of resistance mediating plasmids, a genus, or family of organisms, and the like. The target sequence may, however, also be selected to be specific for one specific marker sequence, e.g. one specific type of antibiotic resistance gene. In case cancer shall be detected, a nucleic acid sequence comprising a cancer-specific mutation may be the target sequence; as is known in the art, cancer-specific mutations, e.g. coding for a cancer neoepitope, can be detected by a mutation-specific 3' targeting sequence, but also using a non-mutation specific 3' targeting sequence 5' of the targeted mutation and sequencing the product of the method described herein.

The term "template DNA", as used herein, relates to any DNA polynucleotide or mixture of DNA polynucleotides comprising or suspected to comprise at least one marker sequence, and, thus, preferably comprising at least one target sequence. Preferably, the template DNA is comprised in or obtained from a sample, preferably a biological sample, thus, the template DNA preferably comprises a plurality of non-identical DNA polynucleotides. E.g., in case the sample comprises cells, the template DNA may comprise at least thousands, or even millions, of non-identical DNA polynucleotides. The template DNA preferably comprises DNA polynucleotides with a length of at most 500 bp, more preferably at most 200 bp, even more preferably at most 100 bp, still more preferably at most 50 bp. Also preferably, the template DNA has a length of at least 20 bp, more preferably of at least 25 bp, still more preferably at least 30 bp, even more preferably at least 35 bp. Thus, the template DNA preferably has a length of from 20 bp to 500 bp, more preferably of from 25 bp to 300 bp, still more preferably of from 30 to 200 bp. Thus, in case it is known or suspected that the sample comprises on average longer DNAs, these may be fragmented and/or size-selected before use as template DNA. Also preferably, the template DNA comprises, more preferably is, cell-free DNA (cfDNA) isolated from a bodily fluid sample, preferably a blood-derived sample. cfDNA and methods for its isolation are known in the art, e.g. from Aucamp et al. (2018) Biol. Rev. 93, 1649-1683.

As used herein, the term "5' universal sequence" relates to a nucleic acid sequence preferably enabling specific amplification of polynucleotides comprising said 5' universal sequence, e.g. by PCR. As the skilled person understands in view of the description herein, the designation "5'" in the context of the 5' universal sequence relates to the position of the universal sequence within the fusion primer as described herein below relative to the 3' targeting sequence. Also, the 5' universal sequence does not necessarily have to form the 5' end of the fusion primer, i.e. the fusion primer may also comprise further nucleic acid sequences 5' of the 5' universal sequence. Preferably, however, the 5' universal sequence forms the 5' end of the fusion primer. The 5' universal sequence is preferably selected to not be present in the plurality of template DNAs. Thus, in case e.g. a given virus shall be detected in a human subject, the 5' universal sequence may be selected such that it occurs neither in said virus nor in said human subject. Preferably, the 5' universal sequence is selected to not be present in any known naturally occurring DNA, preferably as determined by the state of databases on 01 September 2023. Corresponding sequences are known in the art, e.g. from Heath et al. (2000) J. Med. Genet. 37, 272-280. Thus, the universal sequence preferably comprises, more preferably consists of, the sequence of SEQ ID NO:1 or a sequence at least 80% identical thereto. More preferably, the universal sequence comprises, preferably consists of, the sequence of SEQ ID NO:1.

In concurrence with the above, the term "universal sequence primer", as referred to herein, relates to an oligonucleotide comprising the 5' universal sequence as specified herein above, or a subsequence thereof, wherein said universal sequence primer has the activity of hybridizing to a detection template. Thus, the universal sequence primer preferably enables amplification of at least one detection template under conditions commonly used for such amplification, such as PCR conditions. In accordance, the universal sequence primer comprises the 5' universal sequence at its 3' end. On an exemplary basis, the universal sequence primer may e.g. comprise, more preferably consist of, a sequence of SEQ ID NO: 1.

The term "3' targeting sequence", as referred to herein, relates to a nucleic acid sequence enabling hybridization, preferably specific hybridization, of a polynucleotide to a target sequence as specified herein above. Thus, preferably, the targeting sequence is essentially complementary to the target sequence, more preferably is complementary to the target sequence. Thus, the 3' targeting sequence preferably comprises the essentially reverse complement of the target sequence. As the skilled person understands in view of the description herein, the designation "3'" in the context of the 3' targeting sequence relates to the position of the targeting sequence within the fusion primer relative to the 5' universal sequence, thus, the 3' targeting sequence may in principle be comprised in other polynucleotides referred to herein also in other positions, preferably in any position deemed appropriate by the skilled person. Thus, the 3' targeting sequence may e.g. hybridize to a 5' portion of a resistance gene. Preferably, the 3' targeting sequence allows hybridization of the fusion primer to a target nucleic acid sequence under the annealing conditions of step (c). Also preferably, the 3' targeting sequence comprises a nucleic acid sequence essentially complementary to a target sequence over a stretch of from 14 to 30 nucleotides, preferably of from 15 to 25 nucleotides, more preferably of from 16 to 24 nucleotides. More preferably, the 3' targeting sequence comprises the nucleic acid sequence of any one of SEQ ID NO:2-38, preferably 2-34 and 36-38.

The term "oligonucleotide comprising a 5' universal sequence and a 3' targeting sequence", which may also be referred to as "fusion primer", relates to any polynucleotide, preferably DNA, comprising the indicated elements, i.e. comprising a 5' universal sequence and a 3' targeting sequence. As indicated herein above, the aforesaid designations as 5' and 3' exclusively relate to the relative positioning of elements within the fusion primer. Thus, the 5' universal sequence is placed 5' of the 3' targeting sequence in the fusion primer, but not necessarily at the 5' end of the fusion primer. Also, the 3' targeting sequence is placed 3' of the 5' universal sequence; however, the 3' targeting sequence preferably is the 3' sequence of the fusion primer. Nonetheless, the fusion primer may comprise additional nucleic acid sequences, e.g. 5' of the 5' universal sequence or intervening the 5' universal sequence and the 3' targeting sequence. Such additional sequences may e.g. be a linker sequences, a barcode sequence, or any sequence deemed appropriate by the skilled person. Preferably, any such additional sequence(s) contributes at most 100 bp, more preferably at most 50 bp, even more preferably at most 20 bp, still more preferably at most 10 bp to the total length of the fusion primer. More preferably, the fusion primer essentially consists of, even more preferably consists of, said 5' universal sequence and said 3' targeting sequence. Preferably, the fusion primer comprises, preferably consists of, a nucleic acid sequence of SEQ ID NO:1 as 5' universal sequence and one nucleic acid sequence selected from SEQ ID NO:2 to 38 as 3' targeting sequence.

As referred to herein, the term "elongation template" relates to a template DNA comprising an adapter molecule at least on one end, preferably on both ends. Preferably, the adapter molecules on both ends of the elongation template are identical. An elongation template may be obtained from a template DNA by any method deemed appropriate by the skilled person, e.g. by standard methods of ligating a, completely or partially, double-stranded adapter molecule to a template DNA. Depending on the source of the template DNA, it may be preferable to perform a blunting reaction on the template DNA before the adapter ligation reaction. As the skilled person understands in view of the description herein, the elongation template may be denatured to produce single-stranded DNA (ssDNA), which is included in the term elongation template as referred to herein. Due to the presence of the long adapter oligonucleotide sequence described herein below, single-stranded elongation template DNA assumes a stem-loop structure after renaturation, with the stem loop formed by sequences forming duplexes under much more denaturing conditions than typical PCR primers, such as the fusion primer, the universal sequence primer, and the adapter primer; i.e., e.g. in PCR, the stem will form already at high temperatures not allowing binding of any of said primers.

As used herein, the term "adapter molecule" includes any and all DNA molecules comprising two polynucleotides hybridizing to each other and together forming a hybrid molecule comprising at least one blunt end. Thus, the adapter molecule preferably is suitable for ligation to any blunt-ended DNA, in particular template DNA. Preferably, the oligonucleotide constituting the 5' end of the template DNA after ligation to a dsDNA (the "long adapter oligonucleotide") is selected to comprise a nucleic acid sequence not melting under the hybridization and elongation conditions of step (c). Thus, the long adapter oligonucleotide preferably has a GC content of at least 50%, preferably at least 60%, more preferably at least 65% and/or has a length of at least 35 bp, preferably at least 40 bp, more preferably at least 42 bp, more preferably at least 44 bp. Preferably, the long adapter oligonucleotide has a GC content of from 45% to 70%, even more preferably of from 50% to 70% and/or a length of from 35 bp to 60 bp, preferably of from 40 bp to 50 bp. Thus, the long adapter oligonucleotide preferably has a melting temperature of at least 75°, preferably of at least 80°C, more preferably of at least 85°C. The adapter molecule preferably further comprises a short adapter oligonucleotide forming the 3' end of a dsDNA ligated to the adapter molecule (the "short adapter oligonucleotide"). The short adapter oligonucleotide preferably is complementary to subsequence of the 3' end of the long adapter oligonucleotide, such that the 5' and the short adapter oligonucleotide, when hybridized to each other, form an adapter molecule with one blunt end an one staggered end. Thus, preferably, the short adapter oligonucleotide has essentially the same GC content as the long adapter oligonucleotide, but preferably is substantially shorter, preferably having a length of from 10 bp to 20 bp, preferably of from 11 bp to 15 bp. Preferably, the adapter molecule comprises, more preferably consists of, one oligonucleotide with the nucleic acid sequence of SEQ ID NO:39 as long adapter oligonucleotide, wherein the 3' nucleotide optionally may be phosphothioate nucleotide; and one oligonucleotide with the nucleic acid sequence of SEQ ID NO:40 as short adapter oligonucleotide. Thus, the adapter molecules referred to herein preferably are suppression adapters in principle known in the art.

The term "adapter primer", as referred to herein, relates to any oligonucleotide hybridizing to the long adapter oligonucleotide of the adapter molecule as described herein above. Preferably, the adapter primer has a melting temperature of less than 75°C, more preferably less than 70°C, still more preferably less than 65°C. Thus, the adapter primer preferably has a melting temperature of from 55°C to 75°C, more preferably of from 60°C to 70°C. Thus, the adapter primer typically may comprise a subsequence of the aforesaid long adapter oligonucleotide or a reverse complement thereof, preferably over a length of from 15 bp to 25 bp, preferably of from 20 to 24 bp. In accordance, the adapter primer preferably is suitable for use as an amplification primer in a PCR reaction using elongation templates as specified herein as templates.

As used herein, the term "detection template" relates to a polynucleotide comprising, in the given order from 5' to 3', a 5' universal sequence, a 3' targeting sequence, optionally a further subsequence of a marker sequence comprising the 3' targeting sequence, and the reverse complement of a long adapter oligonucleotide sequence of an adapter molecule. Thus, the detection template can be amplified, e.g., by PCR using a universal sequence primer and an adapter primer, irrespective of the intervening 3' targeting sequence and optional further subsequence of a marker sequence comprising the 3' targeting sequence. Thus, the detection template can preferably be used to amplify target sequences and, if present, a further subsequence of a marker sequence essentially without bias arising from differences in binding of different primers to their target sequences.

The term "providing" is understood by the skilled person to relate to each and every activity making an indicated item available. Thus, "providing at least one detection template" is used herein in a broad sense including any and all means and methods of providing at least one detection template. As the skilled person understands in view of the description herein, the number of detection templates in fact provided by the method will in particular depend on the number of marker sequences in the sample and the number of fusion primers used. I.e., preferably, the number of identical elongation templates produced for a given marker sequence will depend on the number of polynucleotides comprising said marker sequence in the template DNA, and on the number of fusion primer molecules comprising a targeting sequence recognizing the target sequence of the marker sequence used in the method. Also preferably, the number of non-identical elongation templates depends on the number of fusion primers with non-identical targeting sequences used, but also on the number of marker sequences comprising corresponding target sequences, which are comprised in the template DNA. As the skilled person understands in view of the description herein, it may not always be known whether a given sample in fact comprises a marker sequence, so, in such case, providing at least one detection template may be a bona fide attempt at providing a detection template for the case at least one marker sequence is present in the sample.

The term "incubating" is understood by the skilled person and preferably includes maintaining under conditions suitable for the intended reaction to occur. Thus, incubation may include maintenance in the presence of suitable enzymes, ions and their concentrations, at least one buffer at a suitable pH, nucleotides, and/or temperature. Depending on the intended reaction, incubating may also include a change in one or more parameters of incubation; e.g. in PCR, temperature may change for a multitude of times. Specific parameters of incubations are described herein below and in the Examples. Also, e.g. in sample pretreatment, incubation may also comprise contacting a sample with purification beads (e.g. magnetic beads).

The term "elongation", in the context of elongation of a fusion primer along an elongation template is understood by the skilled person. Preferably, the term includes addition of nucleotides in 5' to 3' direction using an elongation template as template, as known to be performed by DNA polymerases. Preferably, the DNA polymerase has strand displacing properties, i.e. preferably can displace the counterstrand, e.g. from a stem structure of the elongation template.

The method comprises step (a) providing a plurality of elongation templates having a stem-loop structure from said template DNA. As detailed herein above, providing a plurality of elongation templates having a stem-loop structure preferably comprises ligating adapter molecules to the template DNA after an optional blunting reaction performed on the template DNA. Optionally, the elongation templates are denatured, e.g. by heating to at least 90°C, preferably at least 95°C, in step (a), and allowed to cool; said denaturation may preferably be performed in the presence of the fusion primer(s) provided in step (b). Thus, the elongation templates may be provided as stem-loop structures as described herein above. In accordance, step (a) preferably comprises step (a1) providing fragments of said template DNA; step (a2) incubating said template DNA under conditions causing DNA ends to be blunt ends; and/or step (a3) ligating adapter molecules to the ends of said template DNA.

The method further comprises step (b) providing at least one oligonucleotide (fusion primer) comprising a 5' universal sequence and a 3' targeting sequence. As detailed herein above, one fusion primer may be provided. Preferably, step (b) comprises providing a plurality of fusion primers, wherein the 5' universal sequences of said fusion primers are essentially identical and wherein the 3' targeting sequences of said fusion primers are non-identical; thus, the method preferably is a method for providing a plurality of detection templates for detecting a plurality of marker sequences (multiplex detection). Preferably, the aforesaid plurality of fusion primers comprises at least 2, preferably at least 5, more preferably at least 10, still more preferably at least 20, more preferably at least 50, non-identical targeting sequences; more preferably comprises of from 2 to 1000, preferably of from 2 to 500, more preferably of from 3 to 200, still more preferably of from 4 to 150, more preferably of from 5 to 100, non-identical targeting sequences. Thus, in step (b), a corresponding number of non-identical fusion primers is provided.

The method also comprises step (c) incubating said plurality of elongation templates of step (a) with the least one fusion primer of step (b) under conditions (i) allowing hybridization of said at least one fusion primer to at least one target nucleic acid sequence in an elongation template, if present, and (ii) causing elongation along the elongation template of any fusion primer hybridized to an elongation template. Conditions allowing hybridization of one or more fusion primers are determined by the skilled person according to criteria known in the art. E.g., in case the elongation templates are dsDNA, a step of denaturation to produce at least partially ssDNA may be included. Also, renaturation after such denaturation may be performed in the presence of the fusion primer(s), so as to allow their hybridization (annealing) to the target sequences in the elongation templates, if present. Preferably, step (c) comprises heating a PCR mixture to a temperature suitable to melt template DNA, at least partially, into single strands. Also preferably, step (c) comprises fusion primer annealing after initial heating at a temperature of from 45°C to 70°C, more preferably of from 50°C to 69°C, even more preferably of from 55°C to 68°C, still more preferably of from 60°C to 70°C for a time of from 2 min to 60 min, preferably of from 5 min to 30 min, even more preferably of from 10 min to 25 min. Thus, on an exemplary basis, step (c) may comprise heating a PCR mixture, preferably including a Taq polymerase, comprising the elongation templates and the fusion primer(s) to a temperature of about 95°C for about 5 min, followed by fusion primer annealing at about 66°C for about 20 min, followed by elongation at 72°C for about 5 min. Preferably, step (c) is performed at most 5 times, preferably at most 2 times, most preferably is performed once. Amplification of DNA, preferably, in case step (c) is performed more than once, preferably is performed under conditions only allowing linear amplification of DNA, i.e. preferably under conditions not allowing exponential amplification of DNA. Thus, step (c) is preferably performed in the presence of one or more fusion primers as the only amplification primer(s), more preferably the incubation mixture of step (c) comprises the plurality of elongation templates and the at least one fusion primer as only DNA molecules.

Advantageously, it was found in the work underlying the present invention that by using fusion primers and adapter primers as described, preferably in a suppression PCR setting, a multitude of target DNAs can be amplified and detected essentially without bias for specific target sequences over others, improving multiplex detection. Also, it was found that cfDNA, e.g. from blood, can be used to detect and identify not only the presence of a pathogenic microorganism in a subject, but also of pathogenicity factors, such as antibiotic resistances, thus greatly improving and speeding up diagnosis in medical settings, e.g. in identification the causative agent of an inflammatory process, in particular sepsis, and treatment options thereof.

The definitions made above apply mutatis mutandis to the following. Additional definitions and explanations made further below also apply for all embodiments described in this specification mutatis mutandis.

The present invention also relates to a method for determining at least one marker sequence in a sample, said method comprising

　(A) providing at least one detection template for detecting a marker sequence according to the method according to any one of claims 1 to 26;
　(B) amplifying said at least one detection template by contacting said at least one detection template with (i) an oligonculeotide hybridizing to the 5' universal sequence and (ii) an oligonucleotide hybridizing to a nucleic acid sequence comprised in the 3' end of the elongation templates and comprised in the stem of the stem-loop structure of the elongation templates, wherein the oligonucleotide of (i) preferably is a universal sequence primer and the oligonucleotide of (ii) preferably is an adapter primer;
　(C) determining at least one marker sequence or a subsequence thereof in the amplification products of step (B); and
　(D) thereby determining at least one marker sequence.

The term "amplifying" is understood by the skilled person. Preferably, the term includes each and every method of increasing the amount of one or more polynucleotides fulfilling a set of criteria in a mixture. Preferably, in case the polynucleotide is a detection template, said criteria are hybridization, under appropriate conditions, of an adapter primer to said detection template and of an universal sequence primer its counterstrand. Thus, the amplification preferably is primer-based amplification, preferably PCR amplification. In accordance, the step of amplifying said at least one detection template by contacting said at least one detection template with the indicated oligonucleotides preferably is a step of amplifying said at least one detection template by a method comprising contacting said at least one detection template with the indicated primers.

The term "determining", as used herein, refers to each and every measure to determining the presence, absence, or amount of a marker sequence in a sample. Thus, the term typically encompasses qualitative determination, e.g. determination of the presence of a marker sequence above a pre-determined detection limit, as well as quantitative or semi quantitative determination. The determination is made on the amplification products of step (B); appropriate methods are in principle known to the skilled person and include in particular hybridization methods, such as hybridization to one or more detectably labeled bait oligonucleotide, labelling of the amplification products of step (B) and using the labelled products as a probe, e.g. on a microarray, and the like. Determining may, however, also be performed by sequencing, e.g. by a primerless method such as nanopore sequencing, or by a primer-requiring method using a universal sequence primer, an adapter primer, and/or a primer binding to the target sequence or the targeting sequence as sequencing primer(s); however, also random primers may be used for sequencing. Thus, the sequencing may in particular be a method of next generation sequencing and/or deep sequencing known in the art. Preferably, sequencing is performed by nanopore sequencing, a method in principle known in the art.

The method for determining comprises step (A) providing at least one detection template for detecting a marker sequence according to the method described herein above.

The method for determining further comprises step (B) amplifying said at least one detection template by contacting said at least one detection template with (i) an oligonculeotide hybridizing to the 5' universal sequence and (ii) an oligonucleotide hybridizing to a nucleic acid sequence comprised in the 3' end of the elongation templates and comprised in the stem of the stem-loop structure of the elongation templates, preferably comprised in the adapter molecules. As described herein above, step (B) is preferably performed by PCR using an adapter primer and a universal sequence primer. Thus, the marker sequences or subsequences thereof are preferably amplified essentially without bias caused by the marker sequences or subsequences thereof.

The method for determining further comprises step (C) determining at least one marker sequence or a subsequence thereof in the amplification products of step (B). Preferred methods for determining have been described herein above. As the skilled person understands in view of the description herein, a marker sequence will be deemed identified, i.e. having been present in a sample, if a target sequence and/or at least an other subsequence of the marker sequence can be determined.

The present invention further relates to a method for diagnosing disease in a sample of a subject, said method comprising

(I) determining at least one marker sequence in said sample according to the method described herein above; and
(II) based on the determining in step (I) diagnosing the disease.

The term "diagnosing", as used herein, refers to assessing the probability according to which a subject is suffering from a disease or condition or is at risk of developing a disease or condition. Accordingly, the method preferably provides an aid for diagnosis since it might be necessary to further strengthen or confirm said diagnosis by, e.g., a medical practitioner. In particular, as will be understood by those skilled in the art, such an assessment, although preferred to be, may usually not be correct for 100% of the subjects to be diagnosed. The term, preferably, requires that a statistically significant portion of subjects can be identified as suffering from the disease. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 50%, at least 60%, at least 70%, at least 80%, at least 90%, at least 95%. The p-values are, in an embodiment, 0.1, 0.05, or 0.01. It will be understood, moreover, that the methods described herein essentially provide an aid for diagnosis and may be included into or supplemented by other diagnostic measures. Diagnosing as referred to herein includes monitoring, confirmation, and classification of the relevant disease or its symptoms. Monitoring relates to keeping track of an already diagnosed disease, or a complication, e.g. to analyze the progression or regression of the disease, the influence of a particular treatment on the progression of disease or complications arising during the disease period or after successful treatment of the disease. Confirmation relates to the strengthening or substantiating a diagnosis already established using other indicators or markers. Classification relates to allocating the diagnosis according to the strength or kind of symptoms into different classes, e.g. classes of infectious agents, classes of antibiotic resistance, cancer type and the like. Also preferably, diagnosing comprises identification of the presence of a pathogenic microorganism in a subject. Also preferably, diagnosing comprises identification of the presence of cancer in a subject. As the skilled person understands in view of the description herein, diagnosing the presence of a pathogenic microorganism or a cancer may indeed only identify the presence, but not the location of the pathogenic microorganism or cancer within a subject, so further diagnostic measure may be necessary to identify said location.

Preferably, the disease is caused by a pathogenic microorganism, wherein the term "pathogenic microorganism" includes each and every microorganism causing, obligatorily or facultatively, disease in at least one subject. Thus, the pathogenic microorganism may in particular by a bacterium, a virus, a parasite, or a fungus. Preferred pathogenic microorganisms are those causing inflammatory disease, in particular sepsis. Non-limiting, exemplary pathogenic microorganisms are described herein in the Examples.

The disease to be diagnosed may in principle be any disease in which a marker sequence can be determined in sample from a subject. Thus, the disease preferably is a disease caused by a pathogenic microorganism, the presence of which may be detected by the methods described herein. Thus, the disease preferably is an infection, preferably with a pathogenic microorganism. Preferably, the infection causes an inflammatory disease, i.e. a health state where the body of an infected subject mounts an immune response to a pathogenic microorganism. The infection may be a local infection, e.g. at an infection site, may be tissue or organ specific, e.g. in viral infection of a target organ, as e.g. in hepatitis, or may be generalized, affecting the whole body of a subject. Similarly, the immune response may also be local, e.g. as an abscess, may be tissue or organ specific, e.g. in pneumonia, or may be generalized, such as in severe forms of sepsis, which may cause damage and possibly failure of several organ systems. In the case of disease, the skilled person preferably selects the sample type according to the suspected location of infection, e.g. in case of suspected meningitis, cerebrospinal fluid may be used as a sample; in case of a local infection, e.g. pus or infected tissue may be used; in case of a nasopharynx infection, nasal mucus, saliva, or a nasal lavage fluid may be used, and so forth. Also preferably, in case a site of infection cannot be localized or a sample from an infection site is not easily accessible, a sample type may be used which is suspected to be in fluid connection with the site of infection, such as blood, cerebrospinal fluid, ascites, and the like.

Preferably, the disease is cancer. The term "cancer", as used herein, relates to a disease of an animal, including man, characterized by uncontrolled growth by a group of body cells ("cancer cells"). This uncontrolled growth may be accompanied by intrusion into and destruction of surrounding tissue (infiltration) and possibly spread of cancer cells to other locations in the body (metastasis). Preferably, also included by the term cancer is a recurrence of a cancer (relapse).

Thus, preferably, the cancer is a solid cancer, a metastasis, or a relapse thereof. Preferably, the cancer is selected from the list consisting of acute myeloid leukemia (AML), acute lymphoblastic leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, fibrosarcoma, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor. Preferably, at least a part of the cells forming the cancer comprises at least one mutation, preferably a cancer specific mutation, e.g. coding for a cancer neoepitope.

The term "subject", as used herein, relates to a vertebrate organism, preferably a mammal, even more preferably a livestock or companion animal, such as a chicken, a goose, a duck, a goat, a sheep, a cattle, a pig, a horse, a dog, a cat, a hamster, a rat, a mouse, a hamster, or a guinea pig. Most preferably, the subject is a human. Preferably, the subject is known or suspected to suffer from a disease as specified herein above, preferably sepsis.

The present invention also relates to a method of treating and/or preventing disease in a subject, said method comprising

(i) diagnosing disease according to the method described herein above; and
(ii) administering at least one treatment and/or prevention measure to said subject; and
(iii) thereby treating and/or preventing the disease.

The terms "treating" and "treatment" refer to an amelioration of the diseases or disorders referred to herein or the symptoms accompanied therewith to a significant extent. Said treating as used herein also includes an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools described herein above. Preferably, the treatment shall be effective for at least 10%, at least 20% at least 50%, at least 60%, at least 70%, at least 80%, or at least 90% of the subjects of a given cohort or population. The specific methods of treatment are determined by the medical practitioner depending on the disease diagnosed. In case of an infection with a pathogenic microorganism, treatment may in particular comprise administration of one or more antiviral, antibiotic, antiparasitic, and/or antifungal agent. Preferably, treating sepsis comprises administration of at least one antibiotic for which no resistance was determined in a sample from the subject. Also preferably, treatment of sepsis comprises reducing mortality from sepsis.

The term "preventing" refers to retaining health with respect to the diseases or disorders referred to herein for a certain period of time in a subject. It will be understood that the said period of time may be dependent on the preventive treatment administered and individual factors of the subject discussed elsewhere in this specification. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Whether a portion is statistically significant can be determined without further ado by the person skilled in the art using various well known statistic evaluation tools discussed elsewhere in this specification. Preferably, a cohort or population of subjects is envisaged in this context, which normally, i.e. without preventive measures according to the present invention, would develop a disease or disorder as referred to herein. Thus, preventing, as referred to herein, includes diagnosing disease, e.g. an infection, at a stage where severe disease has not developed. I.e., on an exemplary basis, an infection may be diagnosed before sepsis develops, such that sepsis may be prevented.

The present invention also relates to a composition of matter comprising at least one detection template produced or producible according to the method for providing at least one detection template described herein. The present invention also relates to a composition of matter comprising at least one detection template and a plurality of elongation templates having a stem-loop structure.

The composition of matter comprises the indicated compounds and may otherwise have any composition deemed appropriate by the skilled person. Thus, the composition of matter may in particular be a diagnostic composition, e.g. an intermediate composition produced during a diagnostic method, preferably the one described herein above.

The present invention also relates to a device comprising the composition of matter as described herein above.

As used herein, the term "device" includes any and all devices comprising the components specified. Preferably, the device is adapted to perform a method as specified herein. Thus, the device preferably comprises an incubation unit for performing at least step (c) of the method for providing at least one detection template described herein and/or for performing step (C) and/or (D) of the method for determining at least one marker sequence described herein above. The device may further comprise a microprocessor comprising tangibly embedded executable instructions for performing at least one step of a method as specified herein. Thus, the device may in particular be a PCR device and/or a sequencing device. The results of a method may be given as output of raw data which need interpretation by a technician. Preferably, the output of the device is, however, processed, i.e. evaluated, raw data, the interpretation of which does not require a technician.

The present invention also relates to a kit comprising a plurality of fusion primers comprising a 5' universal sequence and a 3' targeting sequence, a universal primer, and an adapter primer.

The term "kit", as used herein, refers to a collection of the aforementioned compounds, means or reagents which may or may not be packaged together. The components of the kit may be comprised by separate vials (i.e. as a kit of separate parts) or provided in a single vial, e.g. as a composition as specified herein above. The housing of the kit in an embodiment allows translocation of the compounds of the kit, in particular common translocation; thus, the housing may in particular be a transportable container comprising all specified components. Moreover, it is to be understood that the kit of the present invention may be used for practicing the methods referred to herein above. It is, in an embodiment, envisaged that all components are provided in a ready-to-use manner for practicing the methods referred to above. Further, the kit preferably contains instructions for carrying out said methods. The instructions can be provided by a user's manual on paper or in electronic form. For example, the manual may comprise instructions for interpreting the results obtained when carrying out the aforementioned methods using the kit. Preferably, the kit comprises further compounds, such as a reaction buffer, a hybridization solution, a lysis buffer, and the like. Preferably, the kit is adapted for use in a method of the present invention, more preferably is adapted to comprise all reagents required to perform said method or methods.

The present invention also relates to a fusion primer comprising a 5' universal sequence and a 3' targeting sequence, a universal primer, and an adapter primer for use in diagnosing disease; and to a use of a fusion primer comprising a 5' universal sequence and a 3' targeting sequence, a universal primer, and an adapter primer the manufacture of a diagnostic.

Also, the present invention relates to a fusion primer comprising a 5' universal sequence and a 3' targeting sequence, a universal primer, and an adapter primer for use in diagnosing disease, preferably inflammatory disease, in particular sepsis, and/or cancer; and to a use of a fusion primer comprising a 5' universal sequence and a 3' targeting sequence, a universal primer, and an adapter primer for the manufacture of a diagnostic for diagnosing disease.

In view of the above, the following embodiments are particularly envisaged:

Embodiment 1: A method for providing at least one detection template for detecting a marker sequence in a sample comprising template DNA, said method comprising

(a) providing a plurality of elongation templates having a stem-loop structure from said template DNA;
(b) providing at least one oligonucleotide (fusion primer) comprising a 5' universal sequence and a 3' targeting sequence;
(c) incubating said plurality of elongation templates of step (a) with the least one fusion primer of step (b) under conditions (i) allowing hybridization of said at least one fusion primer to at least one target nucleic acid sequence in an elongation template, if present, and (ii) causing elongation along the elongation template of any fusion primer hybridized to an elongation template;
(d) removing the fusion primer from the reaction products of step (c); and
(e) thereby providing a detection template for detecting a marker sequence in a sample.

Embodiment 2: The method of embodiment 1, wherein said method is a method for providing a plurality of detection templates for detecting a plurality of marker sequences, and wherein step (b) comprises providing a plurality of fusion primers, wherein the 5' universal sequences of said fusion primers are essentially identical and wherein the 3' targeting sequences of said fusion primers are non-identical.

Embodiment 3: The method of embodiment 1 or 2, wherein said plurality of fusion primers comprises at least 2, preferably at least 5, more preferably at least 10, still more preferably at least 20, more preferably at least 50, non-identical targeting sequences.

Embodiment 4: The method of embodiment 2 or 3, wherein said plurality of fusion primers comprises of from 2 to 1000, preferably of from 2 to 500, more preferably of from 3 to 200, still more preferably of from 4 to 150, more preferably of from 5 to 100, non-identical targeting sequences.

Embodiment 5: The method of any one of embodiments 1 to 4, wherein the stem of the stem-loop structure of elongation templates is formed by adapter molecules ligated to said template DNA.

Embodiment 6: The method of any one of embodiments 1 to 5, wherein step (a) comprises step (a1) providing fragments of said template DNA.

Embodiment 7: The method of any one of embodiments 1 to 6, wherein step (a) comprises step (a2) incubating said template DNA under conditions causing DNA ends to be blunt ends.

Embodiment 8: The method of any one of embodiments 1 to 7, wherein step (a) comprises step (a3) ligating adapter molecules to the ends of said template DNA.

Embodiment 9: The method of embodiment 8, wherein said adapter molecules are suppression adapters comprising nucleic acid sequences not melting under the hybridization and elongation conditions of step (c).

Embodiment 10: The method of any one of embodiments 1 to 9, wherein said elongation templates comprise fragmented DNA, preferably cfDNA.

Embodiment 11: The method of any one of embodiments 1 to 10, wherein said 5' universal sequence is selected to not be present in the plurality of elongation templates.

Embodiment 12: The method of any one of embodiments 1 to 11, wherein said 5' universal sequence is selected to not be present in any known naturally occurring DNA.

Embodiment 13: The method of any one of embodiments 1 to 12, wherein said 5' universal sequence comprises the nucleic acid sequence of SEQ ID NO:1.

Embodiment 14: The method of any one of embodiments 1 to 13, wherein said 3' targeting sequence is essentially complementary to a target sequence comprised in a marker sequence.

Embodiment 15: The method of any one of embodiments 1 to 14, wherein said 3' targeting sequence allows hybridization of the fusion primer to a target nucleic acid sequence under the annealing conditions of step (c).

Embodiment 16: The method of any one of embodiments 1 to 15, wherein said 3' targeting sequence comprises a nucleic acid sequence essentially complementary to a target sequence with a length of from 14 to 30 nucleotides, preferably of from 15 to 25 nucleotides, more preferably of from 16 to 24 nucleotides.

Embodiment 17: The method of any one of embodiments 1 to 16, wherein said 3' targeting sequence comprises the nucleic acid sequence of any one of SEQ ID NO:2 to 38.

Embodiment 18: The method of any one of embodiments 1 to 17, wherein said fusion primer consists of said 5' universal sequence and said 3' targeting sequence.

Embodiment 19: The method of any one of embodiments 1 to 18, wherein said marker sequence is a nucleic acid sequence specific for a pathogenic microorganism or a group of pathogenic microorganisms, for an antibiotic resistance gene or a group of antibiotic resistance genes, preferably wherein said pathogenic microorganism is a bacterium, a virus, a unicellular parasite, or a fungus.

Embodiment 20: The method of any one of embodiments 1 to 19, wherein said conditions allowing hybridization in step (c) (i) comprise incubation at a temperature of from 50°C to 75°C, preferably of from 60°C to 70°C, more preferably of about 65°C.

Embodiment 21: The method of any one of embodiments 1 to 20, wherein said conditions causing elongation in step (c)(ii) comprise incubation in the presence of a DNA polymerase, DNA nucleotides, and optionally a buffer and magnesium ions.

Embodiment 22: The method of any one of embodiments 1 to 21, wherein said DNA polymerase is a thermostable DNA polymerase.

Embodiment 23: The method of any one of embodiments 1 to 22, wherein step (c) is performed at most 5 times, preferably at most 2 times

Embodiment 24: The method of any one of embodiments 1 to 23, wherein step (c) is performed once.

Embodiment 25: The method of any one of embodiments 1 to 24, wherein the incubation mixture of step (c) comprises the plurality of elongation templates and the at least one fusion primer as only DNA molecules.

Embodiment 26: The method of any one of embodiments 1 to 25, wherein the plurality of elongation templates is not preamplified.

Embodiment 27: A method for determining at least one marker sequence in a sample, said method comprising

(A) providing at least one detection template for detecting a marker sequence according to the method according to any one of embodiments 1 to 26;
(B) amplifying said at least one detection template by contacting said at least one detection template with (i) an

oligoncu, leotide hybridizing to the 5' universal sequence and (ii) an oligonucleotide hybridizing to a nucleic acid sequence comprised in the 3' end of the elongation templates and comprised in the stem of the stem-loop structure of the elongation templates, wherein the oligonucleotide of (i) preferably is a universal sequence primer and the oligonucleotide of (ii) preferably is an adapter primer;

(C) determining at least one marker sequence or a subsequence thereof in the amplification products of step (B); and

(D) thereby determining at least one marker sequence.

(E) The method of embodiment 27, wherein amplifying in step (B) comprises contacting said detection template to a first primer oligonucleotide comprising the 5' universal sequence or a subsequence thereof (universal sequence primer) and to a second primer oligonucleotide hybridizing to said nucleic acid sequence comprised in the 3' end of the elongation templates and comprised in the stem of the stem-loop structure of the elongation templates (adapter primer).

Embodiment 28: The method of embodiment 27 or 28, wherein said first primer oligonucleotide comprises the nucleic acid sequence of SEQ ID NO:38.

Embodiment 29: The method of any one of embodiments 27 to 29 wherein said second primer oligonucleotide comprises the nucleic acid sequence of SEQ ID NO:1.

Embodiment 30: The method of any one of embodiments 27 to 30, wherein step (C) comprises sequencing at least a fraction of the amplification products of step (B).

Embodiment 31: The method of embodiment 31, wherein said sequencing is nanopore sequencing.

Embodiment 32: A method for diagnosing disease in a sample of a subject, said method comprising

(I) determining at least one marker sequence in said sample according to the method according to any one of embodiments 27 to 32; and

(II) based on the determining in step (I) diagnosing the disease.

Embodiment 33: The method of embodiment 33, wherein said disease is a disease caused by a pathogenic microorganism, preferably is an inflammatory disease, more preferably is sepsis, or is cancer.

Embodiment 34: The method of embodiment 33 or 34, wherein in step (II) the disease is diagnosed in case at least one marker sequence indicative of said disease is determined in step (I).

Embodiment 35: The method of any one of embodiments 33 to 35, wherein said method comprises performing further diagnostic measures.

Embodiment 36: A method of treating and/or preventing disease in a subject, said method comprising

(i) diagnosing disease according to the method according to any one of claims 33 to 37; and

(ii) administering at least one treatment and/or prevention measure to said subject; and

(iii) thereby treating and/or preventing the disease.

Embodiment 37: The method of embodiment 36, wherein the disease is sepsis and said at least one treatment comprises administering at least one antibiotic to said subject.

Embodiment 38: The method of embodiment 37, wherein said marker sequence is a sequence indicative of an antibiotic resistance gene and wherein at least one antibiotic administered is selected to overcome resistance mediated by said resistance gene.

Embodiment 39: The method of embodiment 36 or 37, wherein said marker sequence is a sequence indicative of a pathogenic microorganism or a group of pathogenic microorganisms and said treatment and/or prevention measure is selected to be effective against said pathogenic microorganism or a group of pathogenic microorganisms.

Embodiment 40: The method of any one of embodiments 1 to 40, wherein said sample comprises a plurality of non-identical template DNAs.

Embodiment 41: The method of any one of embodiments 1 to 41, wherein said sample is known or suspected to comprise at least one marker sequence.

Embodiment 42: The method of any one of embodiments 1 to 42, wherein said sample is a biological sample, preferably a blood or blood-derived sample.

Embodiment 43: The method of any one of embodiments 1 to 43, wherein said sample comprises cell-free DNA (cfDNA).

Embodiment 44: The method of any one of embodiments 1 to 44, wherein said sample is a sample of a subject.

Embodiment 45: The method of embodiment 45, wherein said subject suffers or is suspected to suffer from sepsis.

Embodiment 46: A composition of matter comprising at least one detection template produced or producible according to the method of any one of embodiment 1 to 26.

Embodiment 47: A composition of matter comprising at least one detection template and a plurality of elongation templates having a stem-loop structure.

Embodiment 48: The composition of matter of embodiment 47 or 48 having at least one feature of any one of embodiments 1 to 46.

Embodiment 49: A device comprising the composition of matter according to any one of embodiments 47 to 49.

Embodiment 50: The device of embodiment 50, wherein said device is a diagnostic device.

Embodiment 51: The device of embodiment 50 or 51, further comprising a thermocycling unit and/or a sequencing unit.

Embodiment 52: A kit comprising a plurality of fusion primers comprising a 5' universal sequence and a 3' targeting sequence, a universal primer, and an adapter primer.

Embodiment 53: The kit of embodiment 53, wherein said fusion primers, said universal primer and/or said adapter primer have a feature according to any one of embodiments 1 to 46.

Embodiment 54: A fusion primer comprising a 5' universal sequence and a 3' targeting sequence, a universal primer, and an adapter primer for use in diagnosing disease.

Embodiment 55: Use of a fusion primer comprising a 5' universal sequence and a 3' targeting sequence, a universal primer, and an adapter primer the manufacture of a diagnostic.

Embodiment 56: A fusion primer comprising a 5' universal sequence and a 3' targeting sequence, a universal primer, and an adapter primer for use in diagnosing sepsis.

Embodiment 57: Use of a fusion primer comprising a 5' universal sequence and a 3' targeting sequence, a universal primer, and an adapter primer for the manufacture of a diagnostic for diagnosing disease.

Embodiment 58: Use of a fusion primer comprising a 5' universal sequence and a 3' targeting sequence, a universal primer, and an adapter primer for diagnosing disease.

Embodiment 59: The use of embodiment 59, wherein said use comprises performing the steps of any one of embodiments 1 to 36.

All references cited in this specification are herewith incorporated by reference with respect to their entire disclosure content and the disclosure content specifically mentioned in this specification.

Figure Legends

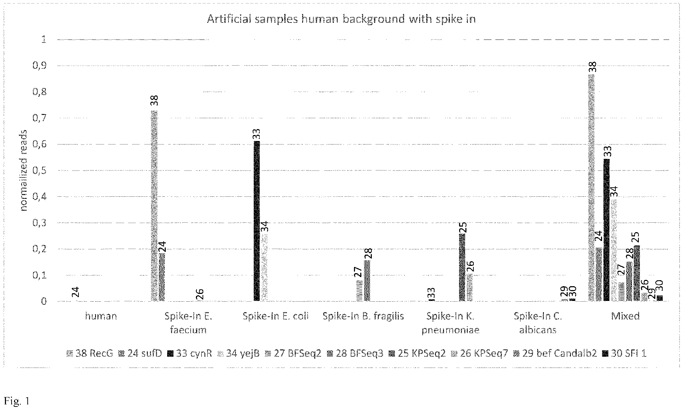

Fig. 1: Detection of spiked-in bacterial DNA in samples. y-axis: read count of specific sequences, normalized to the read counts of the internal human control amplicon IgKnumbers indicate the corresponding primer SEQ ID NOs.

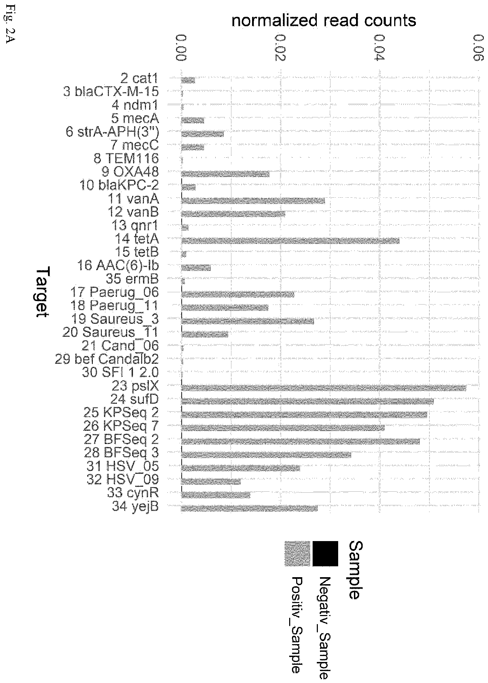

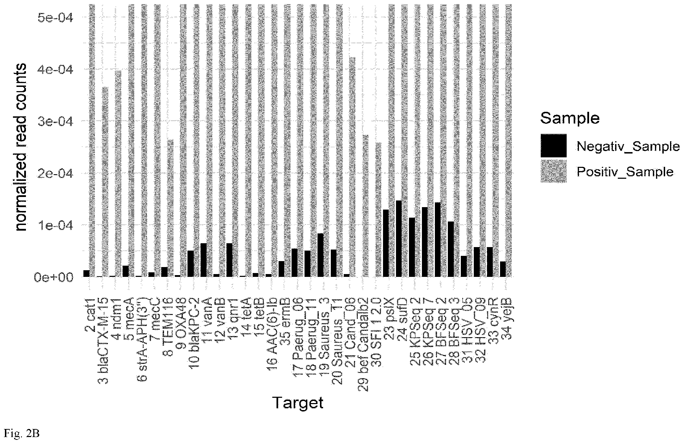

Fig. 2: Technical validation of primer panel on Spike-In positive and negative sample. y-axis: read count of specific sequences, normalized to total number of filtered reads numbers indicate the corresponding primer SEQ ID NOs. (A) shows normalized reads in normal scale, (B) shows the same data with scale adapted to enlarge low values, i.e. values of positive sample are cut off at the top.

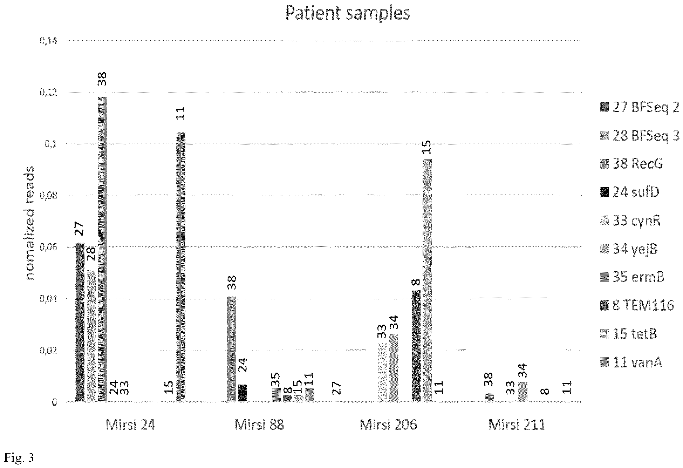

Fig. 3: Detection of causative pathogens and AMRs in septic patient samples; y-axis: read count of specific sequences, normalized to the read counts of the internal human control amplicon IgK numbers indicate the corresponding primer SEQ ID NOs. Mirsi 24, Mirsi 88, Mirsi 206, and Mirsi 211 are patient pseudonyms.

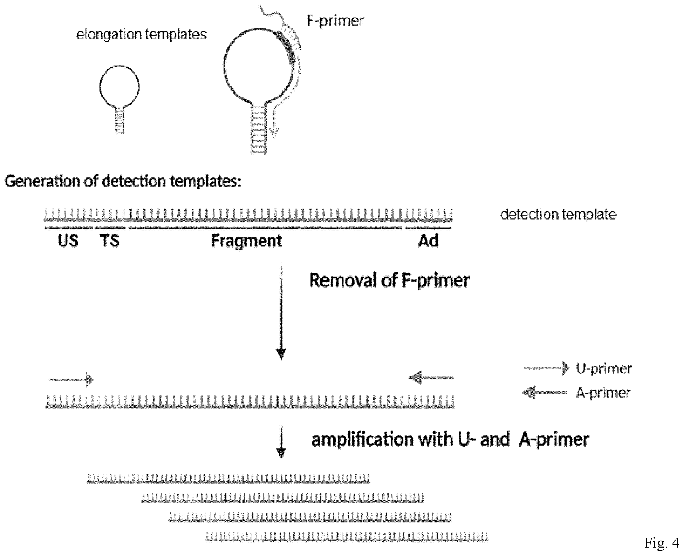

Fig. 4: SUPSETS steps: After suppression adapters are ligated to both ends of a fragment, the elongation reaction follows. The fusion primer (F-primer), consisting of target specific sequence (TS) and a 5' universal sequence (US), binds to its target region and generates a detection template. All recognized sequences form templates, which have a 5' universal sequence and 3' adapter sequence (Ad). In the elongation step, megaplexing can take place and n sequences can be recognized with a number of n primers. All non-recognized elongation templates are suppressed by hybridization of suppression adapters. In the amplification step, an amplification of all detection templates occurs with the same pair of primers, the universal primer (U primer) and the adapter primer (A primer).

The following Examples shall merely illustrate the invention. They shall not be construed, whatsoever, to limit the scope of the invention.

Example 1: Preparing cfDNA for Suppression PCR-based selective enrichment sequencing (SUPSETS) experiments

- Isolation of cfDNA

Isolation of cfDNA from septic patients was performed in an earlier study from the in-vitro diagnostic innovation field (Grumaz et al. (2019) Crit. Care Med. 47, e394-e402.). Comparable for healthy control samples, plasma from healthy individuals was centrifuged for 10 min and 16,000xg at 4 °C. Afterwards, 1.1 mL of plasma was collected from each sample and transferred in a fresh 1.5 mL DNA LoBind tube. If necessary, the sample volume was filled up with freshly prepared 1x PBS to 1.1 mL in total. After mixing the samples by vortexing and a short centrifugation, cfDNA was isolated by the QIAsymphony® SP DNA Preparation System and the QIAsymphony® DSP Circulating DNA Kit according to the manufacturer's advice. In the end, cfDNA was eluted in 60 μL of nuclease-free water and stored at -80 °C until further use. The quality of cfDNA isolation was checked by DNA concentration measurements and fragment analysis

- End-repair of cfDNA

Suppression adapters were ligated to the ends of DNA, either fragmented genomic DNA or isolated cfDNA. This step requires blunt-end DNA fragments, which are not always present in isolated cfDNA. To produce blunt-end cfDNA fragments, the NEB Quick Blunting Kit was used according to the manufacturer's advice. In short, a reaction batch was prepared according to Table 1 and incubated for 20 min at RT. After heat inactivation of the enzyme for 10 min at 70 °C, the end-repaired cfDNA was ready to use for following adapter ligation step.

Table 1: Protocol for the end-repair of cfDNA (blunting):

| | |
|---|---|
| **cfDNA (up to 5 μg)** | 19 μL |
| **10x Blunting Buffer** | 2.5 μL |
| **1 mM dNTP Mix** | 2.5 μL |
| **Blunt Enzyme Mix** | 1 μL |

Example 2: Suppression adapter hybridization

A pseudo-double-stranded adapter, so-called suppression adapter, was annealed to the ends of the DNA fragments. This suppression adapter consisted of a long (Adapter_long, SEQ ID NO:36 with a phophotioate nucleotide as the 3' nucleotide) and short (Adapter short, SEQ ID NO:37) oligonucleotide which were hybridized. To prepare the suppression adapter in a final concentration of 20 μM, equimolar amounts (2 nmol) of both adapter oligonucleotides were mixed and the reaction according to Table 2 was prepared:

Table 2: Hybridization protocol for suppression adapter

| | |
|---|---|
| **100 μM Adapter_short** | 20 μL |
| **100 μM Adapter_long** | 20 μL |
| **10x T4 Ligase Mix** | 10 μL |
| **Nuclease-free water** | 50 μL |

The reaction mixture was incubated at 95 °C for 3 min and cooled down to RT overnight. Suppression adapters were stored at -20 °C until continuation with adapter ligation.

Example 3: Ligation of suppression adapters

Suppression adapters were ligated to blunt-end cfDNA fragments or enzyme-digested genomic DNA. For adapter ligation, 20 fmol of hybridized adapter were added to 400 U of T4 ligase and blunt-end DNA fragments in a DNA to adapter ratio of 1:20. Nuclease-free water was added to adjust the 10x ligation buffer to a one-fold concentration (see Table 14). The reaction was incubated either for 2 h at RT or 16 °C over night. After heat-inactivation at 65°C for 10 min, adapter-ligated DNA was purified with AMPure XP Beads and DNA concentration was measured by Qubit.

Successful suppression adapter ligation was validated by comparing the reaction before and after the ligation reaction by gel electrophoresis (Fragment Analyzer, Agilent): a size-shift of approximately 50 - 80 bp indicated successful suppression adapter ligation.

Table 3: Exemplary suppression adapter ligation mixture

| DNA | 600 - 700 ng |
|---|---|
| 20 μM Suppression Adapter | 4 μL |
| 10x Ligase Buffer | 4 μL |
| T4 Ligase | 2 μL |
| Nuclease-free water | Up to 40 μL |

Example 4: Elongation reaction

For the elongation reaction, Fusion primers (F-primers) against target-specific sites were designed. The reaction for a typical elongation mix is depicted in Table 4:

Table 4: Conditions for one elongation reaction

| 10x AmpliTaq Buffer | 2.5 μL |
|---|---|
| 25 mM magnesium chloride | 2.5 μL |
| 10 mM dNTPs | 1 μL |
| Adapter ligated DNA | Genomic DNA: 20 ng<br>cfDNA: 7.5 - 20 ng |
| 10 μM Fusion Primer (per Primer) | Single: 0.75 μL ($\triangleq$ 0.3 μM)<br>Multiplex: 0.25 - 0.3 μL ($\triangleq$ 0.1 - 0.12 μM) |
| AmpliTaq Gold 360 Polymerase | 0.25 μL $\triangleq$ 1.25 U |
| Nuclease-free water | Up to 25 μL |

To determine the genome equivalents spiked in the reaction, the following formula was used:

$$m\,[pg] = \frac{genome\ size\,[bp] \cdot M_w\,\left[\frac{g}{mol}\right] \cdot GE \cdot 10^{12}\,\left[\frac{pg}{g}\right]}{N\,\left[\frac{molecules\ or\ bp}{mol}\right]}$$

$$= \frac{genome\ size \cdot 650 \cdot GE \cdot 10^{12}}{6.022 \cdot 10^{23}}$$

For the elongation, the following conditions were used:

- Denaturation: 95 °C 5 min
- Annealing: 66 °C 20 min
- Extension: 72 °C 5 min
- Hold: 4 °C ∞

Afterwards, the elongation products were purified with 1.6x AMPure XP Beads to reaction volume to remove excessive F-primers and was further used for the amplification reaction by PCR.

Example 5: Amplification via polymerase chain reaction (PCR)

Following the elongation reaction with F-primers, all generated templates were be amplified by PCR with the same pair of primers with the 5' universal sequence and the 3' adapter sequence. For one PCR amplification, the following reaction mix was prepared, depicted in Table 5.

Table 5: Mix for PCR amplifications

|  | SUPSETS PCR |
|---|---|
| **10x AmpliTaq Buffer** | 2.5 μL |
| **25 mM magnesium chloride** | 2.5 μL |
| **10 mM dNTPs** | 1 μL |
| **Elongation product** | 14 μL |
| **Double-digested gDNA** | - |
| **10 μM US-FW primer** | 0.5 μL ≙ 0.2 μM |
| **10 μM Ad-rev primer** | 0.5 μL ≙ 0.2 μM |

For the PCR, the following conditions were used:

| | | | |
|---|---|---|---|
| **Initial Denaturation:** | **95 °C** | **4 min** | |
| **Denaturation:** | **95 °C** | **20 see** | |
| **Annealing:** | **65 °C** | **30 see** | **38 cycles** |
| **Extension:** | **72 °C** | **30 see** | |
| **Final Extension:** | **72 °C** | **5 min** | |
| **Hold:** | **4 °C** | **∞** | |

Following the amplification the sample was optionally purified using 1,6 x AMP XP Beads. Afterwards, the PCR amplification reaction was analyzed using a Fragment Analyzer device for amplicon sizes and/or was further used for Nanopore sequencing.

Example 6: Sequencing (nanopore sequencing)

With the amplified DNA library, sequencing experiments were performed on the MinION Mk1B or MinION Mk1C device either with a MinION Flow Cell or a Flongle flow cell each type R9.4.1. Before sequencing, all used flow cells were tested for pore activity with the MinKNOW software. Priming of the flow cell was performed according to the manufacturer's instructions, depending on using a MinION Flow Cell or Flongle flow cell. In the meantime, the sequencing library was prepared according to the ONT ligation sequencing-based protocol for a MinION Flow Cell or Flongle flow cell, differing only in the amounts of reagents and DNA library. The sequencing experiments were carried out for 3 h or 24 h and afterwards analyzed.

Example 7: Results

7.1 Figure 1

Spike-In samples 4,000 GE of respective pathogen to 6,000 GE human. Shown are a human sample with 6,000 GE of suppression adapter-ligated DNA on the left, five samples with spiked-in 4,000 GE of the respective pathogenic DNA (Spike-in E. coli, Spike-in E. faecium, Spike-In B. fragilis, Spike-In K. pneumoniae and Spike-In C. albicans) to the human DNA and a mixed sample with human DNA and 4,000 GE of each pathogen DNA spiked-in on the right.

After the SUPSETS workflow and sequencing for 21 h on the Mk1B sequencing device with a MinION Flongle flow cell, data were processed. Maximum coverage per target region were normalized to the human control amplicon IgK.

As can be seen in Figure 1, bacteria-specific sequences could be detected, while non-specific background reads were low. Little to no normalized reads were detected for the human or any non-targeted Spike-In pathogen sample. Both *E. faecium* amplicons displayed relatively high normalized read counts. The same held true for both *E. coli* amplicons: strong and visible signals for the *E. coli* respective primer SEQ-ID 33 and 34.

Both amplicons corresponding to *B. fragiles* were obtained in the Spike-in B. fragilis sample as well (see SEQ-IDs 27 and 28). The same observation could be made in Figure 20 for the detection of *K. pneumoniae* with the SEQ-IDs 25 and 26. Here, a relatively small signal for primer 33 was detected, but in comparison to primers 25 and 25, a clear difference is visible. For the *C. albicans* representing amplicons, little unspecific signal was received. Also the normalized reads for *C. albicans* corresponding primers SEQ-ID 29 and 30, the signals were low but detectable. The Mixed sample in figure 1 shows, that all amplicons could be resolved in the sample. While some primers displayed a strong signal, others were weaker but still visible comparing to the low non-specific signals received within this experiment.

7.2 Figure 2

To test the performance of the megaplex approach a spiked-in sample containing all targeted pathogens and plasmids holding the AMR target sequences with human background was performed. To rule out unspecific binding of primers against the human genome a negative sample with the complete primer set of 33 primers was performed without the addition of pathogen or AMR-plasmid DNA.

As input for the positive sample, 4,000 genome equivalents (GE) of all pathogens (*E. coli, E. faecium, B. fragilis, K. pneumonia, C. albicans, P. aeruginosa, S. aureus,* HSV-1) as well as of the resistance plasmids were spiked-in 6,000 GE of human background. For the negative control, the complete primer panel was tested without any pathogens or AMR-plasmid spiked-in but 6,000 GE of human DNA. SUPSETS workflow was performed and samples were sequenced on a Mk1B device with a Flow Cell for 22 hours. After data were processed, maximum coverage per target region were normalized to the total filtered reads of the respective sample.

The results show, that all primers gained a traceable amplification in the positive (all spiked-in) sample. Some basic amplicons could been detected in the negative sample for all Primers besides SEQ-ID 29 and 30. Results differ in at least one magnitude of order between negative and positive sample, in most cases two to three magnitudes of order. A clear difference between signals obtained in the positive samples in comparison to the negative samples can be seen.

7.3 Figure 3

Detection of causative pathogens and AMRs was performed as described above in septic patient samples. Sequencing was performed for 24 h on the Mk1B sequencing device with a MinION Flongle flow cell, data were processed. Maximum coverage per target region were normalized to the read counts of human control amplicon IgK. Only target sequences are depicted that generated at least one read for any sample - not detected sequences are not shown.

According to the Illumina sequencing results, the expectations for the Mirsi 24 patient samples were that signals should be detected for *E. coli, E. faecium* and *B. fragilis,* with the highest bacterial burden for *B. fragilis.* As shown in figure 3, both amplicons representing *B. fragilis* (SEQ-ID 27 and 28) were detected. In addition, the *E. faecium* amplicon RecG (SEQ-ID 38) was depicted. *E. coli* normalized reds for primer 33 were barely detected within this experiment, only one. Besides that, the resistance gene vanA showed high normalized read numbers and was detected.

In Mirsi 88, signals for *E. faecium* and human are expected. Normalized reads for *E. faecium* were detected especially for primer with SEQ-ID 38 but also to some extent for SEQ-ID 24. Some lower but detectable normalized signals could be found for resistance genes ermB (35), tetB (15), TEM116 (8) and vanA (SEQ-ID 11).

For Mirsi 206, *E. coli* is expected as only pathogenic burden. In fact, cynR (SEQ-ID 33) and yejB (34) were both detected clearly, respectively. Furthermore, the resistance genes tetB and TEM116 were found with relatively high normalized reads.

In the patient sample Mirsi 211, signals for *E. coli, E. faecium* and *K. pneumoniae* were obtained. No normalized reads were detected for *K. pneumoniae* amplicons. *E. faecium* was detected with amplicon RecG (SEQ-ID 38). For both *E. coli* amplicons, a signal was obtained, especially yejB (SEQ-ID 34) shows visible normalized read numbers. Resistance genes vanA and TEM116 were obtained with small signals.

Example 8: Provision of elongation templates from various sample types

A sample, which may be any sample known or suspected to comprise biological material and/or DNA, is extracted to liberate DNA. Appropriate methods for DNA extraction are selected without further ado by the skilled person depending on the sample material. In case of dilute samples, DNA may be concentrated by precipitation, by binding to particles, e.g. particles compising positive charges, and the like. If it is known or suspected that DNA in the sample is comprised of long polynucleotides, e.g. longer than approx. 200 to 500 bp, the DNA in the sample may be fragmented, e.g. by shearing, restriction enzyme digestion, DNase treatment, and the like. The DNA thus obtained is the template DNA as specified herein. To this template DNA, which may be pretreated to ensure that DNA ends are flush, adapter molecules are ligated as specified above, creating elongation templates.

From the above, the skilled person understands that the methods of the present invention can be applied essentially to any sample material from which DNA can be extracted.

Table 6: Sqeuences used, for targeting sequences (SEQ ID NOs:2 to 38, the respective targets are indicated; AMR: antimicrobial resistance gene.

| SEQ ID NO | Sequence | Designation | indicative of |
|---|---|---|---|
| 1 | TCCGTCTTAGCTGAGTGGCGTA | universal | --- |
| 2 | AAGATGTGGCGTGTTACGG | A cat1_03_FW | AMR |
| 3 | GGAGACGAAACGTTCCG | A _blaCTX-M-15 02 FW short | AMR |
| 4 | ACATCGCTTTTGGTGGC | A_ndm1_02 FW short | AMR |
| 5 | AATCGATGGTAAAGGTTGGC | A_mecA_02_FW | AMR |
| 6 | GCATTCTGACTGGTTGCC | A_strA-APH(3")_01_FW | AMR |
| 7 | CATCACCAGGTTCAACCC | mecC US-FW | AMR |
| 8 | TGAGGCACCTATCTCAGCG | TEM116 2.0 US-FW | AMR |
| 9 | GCACGTATGAGCAAGATGC | OXA48 4.0 US-FW | AMR |
| 10 | TCTGACAACAGGCATGACG | blaKPC-2 2.0 US-FW | AMR |
| 11 | CCAATACCGCACAACCGA | vanA US-FW | AMR |
| 12 | GAAGTGGATCAAATCCGGC | vanB US-FW | AMR |
| 13 | CAATGCCAATCTCAGCGG | qnr1 US-FW | AMR |
| 14 | AGGCAAGCAGGATGTAGC | tetA US-FW | AMR |
| 15 | CAAAGGCTTGGAATACTGAGTG | tetB US-FW | AMR |
| 16 | TCATATCGTCGAGTGGTGG | AAC(6)-Ib 3.0 US-FW | AMR |
| 17 | GATCGAGTCGTTCATCGG | B_Paerug_06_FW | P. aeroginosa |
| 18 | CAGGATATCGCTCAACCG | B_Paerug_11_FW | P. aeroginosa |
| 19 | GTAGGTACATCATCGTTAAGGC | B_Saureus_3_FW | S. aureus |
| 20 | GCGATTATTGTTGCTGAAGG | B_Saureus_11_FW | S. aureus |
| 21 | GGATTGCCTTGTCTAACCC | G_Cand_06_FW | Candida spp |
| 22 | CCATGAAGTCGGAATCGC | A_16s_02_FW | 16S rDNA |
| 23 | GCAATTGTAGAAGGCGTGGC | plsX | E. faecium |
| 24 | AAGACGCACTAGCGAAAGC | sufD US-FW | E. faecium |
| 25 | GTGGTGGAAGTCTACACCG | KPSeq 2 US-FW | K. pneumonice |
| 26 | GGCTATGGGATGATATTGCC | KPSeq 7 US-FW | K. pneumonice |
| 27 | TCGGAAGGAGAGAACATCC | BFSeq 2 US-FW | B. fragilis |
| 28 | TCTATAGATGGCAACACACGG | BFSeq 3 US-FW | B. fragilis |
| 29 | GCTATCGCAAGTTCTACCGG | bef Candalb2 US-FW | C. albicans |
| 30 | CTGCCTCCATACTTTGTAGGC | SFI 1 2.0 | C. albicans |
| 31 | GGTCGAAAGCGTACTTGGC | V HSV 05 FW | HSV-1 |
| 32 | GCAACTCGTAGCAAATAGGC | V_HSV_09_FW | HSV-1 |
| 33 | CAGAACCGCGCTAATT | cynR Test 4 | E. coli |
| 34 | CTCGCGCGATTATCC | yejB Test 1 | E. coli |
| 35 | GAACATCTGTGGTATGGCG | ermB US-FW | AMR |
| 36 | GCTATCGCAAGTTCTACCGG | CaSeq2 | C. albicans |
| 37 | AGAGCAGGACAGCAAGGAC | IgK | human |
| 38 | GTCAATCTGGTCTGCCTCA | RecG | E. faecium |

(continued)

| SEQ ID NO | Sequence | Designation | indicative of |
|---|---|---|---|
| 39 | TGTAGCGT-GAAGACGACAGAAAG GGCGTGGTGCGGAGGGCGG*T | Adapter_long | - |
| 40 | ACCGCCCTCCGC | Adapter_short | - |
| 41 | TGTAGCGTGAAGACGACAGAA | Ad rev | - |

Literature cited:

Aucamp et al. (2018) Biol. Rev. 93, 1649-1683
Broude et al., (1999) Genet. Anal. 15, 51-63
Broude et al. (2001) Proc. Natl. Acad. Sci. U. S. A. 98, 206-211
Dolinet al. (2019) Microbiol. Insights 12, 117863611882508
Eykyn et al. (1990) J. Antimicrob. Chemother 25, 41-58
Grumaz et al. (2019) Crit. Care Med. 47, e394-e402.
Heath et al. (2000) J. Med. Genet. 37, 272-280
Mandel & Métais (1948) Comptes Rendus des Seances la Soc. Biol. ses Fil. 142
Mayr et al. (2010) JAMA 303, 2495-2503
Murray & Masur (2012) Crit. Care Med. 40,3277-3282
Patel, M. (2016) J. Acad. Clin. Microbiol. 18, 74
Seymour et al. (2017) N. Engl. J. Med 376, 2235-2244
US 2004/0126760 A1

**Claims**

1.  A method for providing at least one detection template for detecting a marker sequence in a sample comprising template DNA, said method comprising

    (a) providing a plurality of elongation templates having a stem-loop structure from said template DNA;
    (b) providing at least one oligonucleotide (fusion primer) comprising a 5' universal sequence and a 3' targeting sequence;
    (c) incubating said plurality of elongation templates of step (a) with the least one fusion primer of step (b) under conditions (i) allowing hybridization of said at least one fusion primer to at least one target nucleic acid sequence in an elongation template, if present, and (ii) causing elongation along the elongation template of any fusion primer hybridized to an elongation template;
    (d) removing the fusion primer from the reaction products of step (c), and
    (e) thereby providing a detection template for detecting a marker sequence in a sample.

2.  The method of claim 1, wherein said method is a method for providing a plurality of detection templates for detecting a plurality of marker sequences, and wherein step (b) comprises providing a plurality of fusion primers, wherein the 5' universal sequences of said fusion primers are essentially identical and wherein the 3' targeting sequences of said fusion primers are non-identical.

3.  The method of claim 1 or 2, wherein the stem of the stem-loop structure of elongation templates is formed by adapter molecules ligated to said template DNA, wherein said adapter molecules preferably are suppression adapters comprising nucleic acid sequences not melting under the hybridization and elongation conditions of step (c).

4.  The method of any one of claims 1 to 3, wherein said elongation templates comprise cfDNA.

5.  The method of any one of claims 1 to 4, wherein said marker sequence is a nucleic acid sequence specific for a pathogenic microorganism or a group of pathogenic microorganisms, for an antibiotic resistance gene or a group of antibiotic resistance genes, preferably wherein said pathogenic microorganism is a bacterium, a virus, a unicellular parasite, or a fungus.

6. The method of any one of claims 1 to 5, wherein step (c) is performed at most 5 times, preferably at most 2 times, more preferably wherein step (c) is performed once.

7. A method for determining at least one marker sequence in a sample, said method comprising

(A) providing at least one detection template for detecting a marker sequence according to the method according to any one of claims 1 to 6;
(B) amplifying said at least one detection template by contacting said at least one detection template with (i) an oligonculeotide hybridizing to the 5' universal sequence and (ii) an oligonucleotide hybridizing to a nucleic acid sequence comprised in the 3' end of the elongation templates and comprised in the stem of the stem-loop structure of the elongation templates;
(C) determining at least one marker sequence or a subsequence thereof in the amplification products of step (B); and
(D) thereby determining at least one marker sequence.

8. The method of claim 7, wherein amplifying in step (B) comprises contacting said detection template to a first primer oligonucleotide comprising the 5' universal sequence or a subsequence thereof (universal sequence primer) and to a second primer oligonucleotide hybridizing to said nucleic acid sequence comprised in the 3' end of the elongation templates and comprised in the stem of the stem-loop structure of the elongation templates (adapter primer) .

9. The method of claim 7 or 8, wherein step (C) comprises sequencing at least a fraction of the amplification products of step (B).

10. A method for diagnosing disease in a sample of a subject, said method comprising

(I) determining at least one marker sequence in said sample according to the method according to any one of claims 7 to 9; and
(II) based on the determining in step (I) diagnosing the disease.

11. The method of claim 10, wherein said disease is a disease caused by a pathogenic microorganism, preferably is an inflammatory disease, more preferably is sepsis, or is cancer.

12. The method of any one of claims 1 to 11, wherein said sample is a biological sample, preferably a blood or blood-derived sample.

13. A composition of matter comprising at least one detection template and a plurality of elongation templates having a stem-loop structure.

14. A device comprising the composition of matter according to claim 13.

15. A kit comprising a plurality of fusion primers comprising a 5' universal sequence and a 3' targeting sequence, a universal primer, and an adapter primer.

Fig. 1

Fig. 2A

Fig. 2B

Fig. 3

elongation templates

F-primer

**Generation of detection templates:**

US | TS | Fragment | Ad

detection template

**Removal of F-primer**

U-primer
A-primer

**amplification with U- and A-primer**

Fig. 4

Europäisches
Patentamt

European
Patent Office

Office européen
des brevets

# EUROPEAN SEARCH REPORT

Application Number

EP 23 19 5471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/140298 A1 (NATERA INC [US]) 18 July 2019 (2019-07-18) * figures 1-4; example 2 * ----- | 1,2,4-15 | INV. C12Q1/6806 C12Q1/6855 |
| X | WO 2021/035056 A1 (FLUENT BIOSCIENCES INC [US]) 25 February 2021 (2021-02-25) * figure 2 * ----- | 1,2,4-15 | |
| X | WO 2017/162754 A1 (VIB VZW [BE]; UNIV GENT [BE]) 28 September 2017 (2017-09-28) * claim 1; figure 9 * ----- | 1,2,4-15 | |
| X | WO 2016/040901 A1 (UNIV LELAND STANFORD JUNIOR [US]) 17 March 2016 (2016-03-17) * figure 6 * ----- | 1,2,4-15 | |
| X | WO 2019/166565 A1 (HOFFMANN LA ROCHE [CH]; ROCHE DIAGNOSTICS GMBH [DE] ET AL.) 6 September 2019 (2019-09-06) * figure 4 * ----- | 1,2,4-15 | |
| X | WO 2021/178893 A2 (SINGULAR GENOMICS SYSTEMS INC [US]) 10 September 2021 (2021-09-10) * figure 11 * ----- | 1,2,4-15 | TECHNICAL FIELDS SEARCHED (IPC) C12Q |

The present search report has been drawn up for all claims

| Place of search | Date of completion of the search | Examiner |
|---|---|---|
| The Hague | 19 December 2023 | Aslund, Fredrik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                  

& : member of the same patent family, corresponding
document

EPO FORM 1503 03.82 (P04C01)

3

## ANNEX TO THE EUROPEAN SEARCH REPORT
## ON EUROPEAN PATENT APPLICATION NO.

EP 23 19 5471

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2023

| Patent document cited in search report | | | Publication date | Patent family member(s) | | | Publication date |
|---|---|---|---|---|---|---|---|
| WO 2019140298 | A1 | | 18-07-2019 | CA | 3088891 | A1 | 18-07-2019 |
| | | | | CN | 111699269 | A | 22-09-2020 |
| | | | | EP | 3737773 | A1 | 18-11-2020 |
| | | | | EP | 4245863 | A2 | 20-09-2023 |
| | | | | JP | 7322063 | B2 | 07-08-2023 |
| | | | | JP | 2021510541 | A | 30-04-2021 |
| | | | | JP | 2023139220 | A | 03-10-2023 |
| | | | | US | 2021071246 | A1 | 11-03-2021 |
| | | | | WO | 2019140298 | A1 | 18-07-2019 |
| WO 2021035056 | A1 | | 25-02-2021 | CA | 3152038 | A1 | 25-02-2021 |
| | | | | EP | 4017989 | A1 | 29-06-2022 |
| | | | | US | 2021054369 | A1 | 25-02-2021 |
| | | | | WO | 2021035056 | A1 | 25-02-2021 |
| WO 2017162754 | A1 | | 28-09-2017 | CA | 3017561 | A1 | 28-09-2017 |
| | | | | EP | 3433378 | A1 | 30-01-2019 |
| | | | | EP | 3778918 | A1 | 17-02-2021 |
| | | | | ES | 2914396 | T3 | 10-06-2022 |
| | | | | US | 2019048412 | A1 | 14-02-2019 |
| | | | | WO | 2017162754 | A1 | 28-09-2017 |
| WO 2016040901 | A1 | | 17-03-2016 | CN | 107075730 | A | 18-08-2017 |
| | | | | EP | 3191628 | A1 | 19-07-2017 |
| | | | | ES | 2925014 | T3 | 13-10-2022 |
| | | | | US | 2018251848 | A1 | 06-09-2018 |
| | | | | US | 2021363597 | A1 | 25-11-2021 |
| | | | | WO | 2016040901 | A1 | 17-03-2016 |
| WO 2019166565 | A1 | | 06-09-2019 | CN | 111868257 | A | 30-10-2020 |
| | | | | CN | 111936635 | A | 13-11-2020 |
| | | | | EP | 3759241 | A1 | 06-01-2021 |
| | | | | EP | 3759251 | A1 | 06-01-2021 |
| | | | | EP | 4230748 | A1 | 23-08-2023 |
| | | | | JP | 2021514646 | A | 17-06-2021 |
| | | | | JP | 2021514651 | A | 17-06-2021 |
| | | | | JP | 2022160661 | A | 19-10-2022 |
| | | | | US | 2023183797 | A1 | 15-06-2023 |
| | | | | WO | 2019166530 | A1 | 06-09-2019 |
| | | | | WO | 2019166565 | A1 | 06-09-2019 |
| WO 2021178893 | A2 | | 10-09-2021 | EP | 4114978 | A2 | 11-01-2023 |
| | | | | US | 2021277461 | A1 | 09-09-2021 |
| | | | | US | 2022033895 | A1 | 03-02-2022 |
| | | | | US | 2022119878 | A1 | 21-04-2022 |
| | | | | US | 2022282324 | A1 | 08-09-2022 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

**EP 23 19 5471**

This annex lists the patent family members relating to the patent documents cited in the above-mentioned European search report.
The members are as contained in the European Patent Office EDP file on
The European Patent Office is in no way liable for these particulars which are merely given for the purpose of information.

**19-12-2023**

| Patent document cited in search report | Publication date | Patent family member(s) | | Publication date |
|---|---|---|---|---|
| | | US | 2022325341 A1 | 13-10-2022 |
| | | US | 2023203576 A1 | 29-06-2023 |
| | | WO | 2021178893 A2 | 10-09-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040126760 A1

**Non-patent literature cited in the description**

- **EYKYN et al.** *J. Antimicrob. Chemother*, 1990, vol. 25, 41-58
- **DOLIN et al.** *Microbiol. Insights*, 2019, vol. 12
- **MAYR et al.** *JAMA*, 2010, vol. 303, 2495-2503
- **SEYMOUR et al.** *N. Engl. J. Med*, 2017, vol. 376, 2235-2244
- **PATEL, M.** *J. Acad. Clin. Microbiol.*, 2016, vol. 18, 74
- **MURRAY** ; **MASUR**. *Crit. Care Med.*, 2012, vol. 40, 3277-3282
- **MANDEL** ; **MÉTAIS**. *Comptes Rendus des Seances la Soc. Biol. ses Fil.*, 1948, 142
- **AUCAMP et al.** *Biol. Rev.*, 2018, vol. 93, 1649-1683
- **BROUDE et al.** *Genet. Anal.*, 1999, vol. 15, 51-63
- **BROUDE et al.** *Proc. Natl. Acad. Sci. U. S. A.*, 2001, vol. 98, 206-211
- Current Protocols in Molecular Biology. John Wiley & Sons, 1989, 6.3.1-6.3.6
- **SAMBROOK et al.** Molecular Cloning. Cold Spring Harbor Laboratory, 1989
- Nucleic Acids Hybridization: A Practical Approach. IRL Press at Oxford University Press, 1985
- Essential Molecular Biology: A Practical Approach. IRL Press at Oxford University Press, 1991
- **HEATH et al.** *J. Med. Genet.*, 2000, vol. 37, 272-280
- **GRUMAZ et al.** *Crit. Care Med.*, 2019, vol. 47, e394-e402